# EUROPEAN PATENT SPECIFICATION

(11) **EP 1 210 908 B1**
(45) Date of publication and mention of the grant of the patent: **26.07.2006**
(21) Application number: 01403051.4
(22) Date of filing: 28.11.2001
(51) Int. Cl.: A61B 5/103, A61B 5/107, A61B 5/00

(54) **Process for diagnosing conditions of external body portions and features of products applied thereto**
Verfahren zur Diagnose des Zustandes von äusseren Körperteilen und Merkmalen von darauf aufgebrachten Produkten
Procédé de diagnostic de l'état des parties exterieures corporelles et caractéristiques des produits appliqués aux mêmes parties

(30) Priority: 29.11.2000 US 725049
(43) Date of publication of application: 05.06.2002
(73) Proprietor: L'ORÉAL, 75008 Paris (FR)
(72) Inventor: Bazin, Roland, 91570 Bievres (FR); Doublet, Eric, 94800 Villejuif (FR)
(74) Representative: Tanty, François

(56) References cited:
- WO-A-01/75586
- WO-A-97/29686
- DE-A- 2 353 224
- DE-U- 20 010 292
- GB-A- 2 288 511
- US-A- 4 738 537
- US-A- 5 785 960

## Description

### Field of the Invention

The present invention relates to a process for providing, based on scanned information, a diagnosis of a condition of an external body portion of an individual and/or features of a product applied to the external portion.

### Description of Related Art

Accurate diagnosis of cosmetic and dermatological related conditions often requires consultations with professionals having the requisite level of proper skill and training. In the past, such consultations required the individual seeking advice to travel to a site and conduct a personal meeting with a professional who would visualize the individual's skin condition, for example, sometimes with special instruments, and prescribe a corrective treatment plan involving one or more cosmetic and/or dermatological products.

Recently, advances in technology have led to a number of attempts at obviating the need for some of these personal, face-to-face meetings requiring travel. In particular, some consultants provide remote cosmetic or dermatological consultations where the individual in need of the consultation can be located at a geographic location different from that of the consultant. These attempts have been primarily limited because there has been no easy way of sending all of the necessary information to the remote consultant.

GB 2 288 511 discloses a method and apparatus for use in diagnosing medical conditions, such as skin conditions, where there are visual symptoms. This reference discloses operating either a video camera, a camcorder, or a digital still camera to generate an electrical signal that is digitized, compressed, and transmitted to an expert consultant, such as a dermatologist. This technique, however, suffers from a number of drawbacks and limitations. In particular, it is time consuming to operate the video camera, run the software necessary to digitize and compress the image captured by the camera, and then send the compressed image to the consultant. An even more significant limitation relates to the fact that there is no easy way to standardize the image. For example, incorrect lighting, inferior equipment, non-uniform cameras and software programs, incorrect camera operation, or other variables may produce a video image that does not clearly show all of the details of the original area that was recorded. In some cases, the video image submitted to the remote professional might be either completely unusable or result in an incorrect evaluation being made.

Another more simplistic approach involves a consumer filling out a preestablished questionnaire and then sending the completed questionnaire (via a delivery service or the Internet) to a cosmetic product distributor that suggests one or more cosmetic products after reviewing the information provided on the competed questionnaire. Such a process can provide general information useful in advising about some types of basic cosmetic products, but such information is limited by the level of detail in the description provided by the individual and is, therefore, inadequate for many treatments, especially those that are highly specialized and advanced. In addition, both the completion of the questionnaire by the consumer and the evaluation of completed questionnaire by the cosmetics distributor can be time consuming.

In addition to the limitations associated with current means of remote diagnosis, there are also drawbacks associated with some diagnostic methods used during face-to-face consultations with a professional. For example, to examine certain types of skin conditions, some dermatologists use very specialized photographic equipment to obtain a photograph of a skin region being examined. In using one such professional photographic system, called DERMAPHOT, a uniquely designed camera lens is placed in contact with the skin and light is emitted through the lens before taking a photograph of the skin. This technique, however, suffers from a number of drawbacks and limitations. In particular, it is time consuming to properly set up the system, correctly particular, it is time consuming to property set up the system, correctly operate the camera, and request a service to develop the film. An even more significant limitation relates to the fact that the specialized photographic equipment is very expensive. Further, the resolution of photographic images obtained with such systems is not always acceptable.

WO 97/29686 discloses a device for estimating skin wrinkling comprising position sensing detectors for recording the light reflected by the wrinkled skin.

This document discloses that the light reflected by the skin is detected using a triangulation method or a focalisation method.

In light of the foregoing, there is a need for a process that improves diagnosis.

### SUMMARY OF THE INVENTION

Accordingly, processes consistent with the present invention preferably may obviate one or more of the limitations of the related art. Such processes have advantages in the field of cosmetics and/or dermatology, but may also be used in other areas.

One aspect of the invention includes a process for diagnosing, based on scanned information, one or more conditions of an external body portion and/or one or more features of at least one product applied to the external body portion. The process includes obtaining, with an optical image scanner configured in the form of a scanner for scanning documents, scanned image data relating to one or more characteristics of a non-dermatoglyphic external portion of an individual, and/or at least one product applied to the external portion. An analysis of the at least one characteristic is conducted based on the image data. The process further includes determining a diagnosis of at least one condition of the external portion, and/or at least one feature of the product applied to the external portion.

The characteristics of the external portion that are analyzed are preferably characteristics of non-dermatoglyphic body portions. As used herein, the term "non-dermatoglyphic" relates an external area of the body substantially free of dermatoglyphs, wherein dermatoglyphs are features that do not change as a person ages. For example, dermatoglyphs are located on the inferior surface of the hand in the form of fingerprints and palm lines. Some examples of characteristics of "non-dermatoglyphic" body portion, viewable features of hair strands including roots, viewable features of skin including pigmentations and groups of skin cells, viewable features of fingernails and toe nails, and exteriorly viewable features of teeth.

There are many different characteristics of products that could be analyzed with the process according to the invention. For example, the process could be practiced to analyze product characteristics, such as non-transferability, especially for lipstick and foundation makeup; product coverage (i.e., homogeneity), especially for nail enamel or hair conditioner; brilliancy, especially for nail enamel; coloring, especially for various types of makeup products; greasiness, especially for skin lotions; various interactions between the skin and the product, especially for products designed to make wrinkles less visible and products designed to change transparency of the skin; and thickness or amount of the product on the external portion, especially for hair products such as conditioners.

The external portion could be on many different areas of the body of the individual. For example, the external portion could include an area of the skin of the individual, at least one strand of hair of the individual, at least one fingernail of the individual, at least one toe nail of the individual, and at least one tooth of the individual. When the external portion includes the skin of the individual, the external portion may be located on the hand, foot, arm, leg, torso, and/or face (i.e., lips) of the individual. When the external portion includes at least one strand of hair, the strand may be from the scalp, the eyelashes or the eyebrows.

The scanned image data could be obtained in either a first mode, a second mode, or a combination of the first and second modes. In the first mode, the external portion of the individual is placed in the vicinity of a scanning region of the scanner, and the external portion is scanned with the image scanner to obtain the scanned image data. The external portion of the individual is preferably placed into contact with the scanning region (i.e., the glass window pane) of the scanner. In one embodiment, the scanner is a flat bed scanner and the external portion of the individual is moved into contact with the scanning region. In another embodiment, the scanner is a hand-held scanner and the scanner is moved into contact with the external portion of the individual.

In one example of a process including the first mode of obtaining scanned image data, the external portion includes skin of the individual and the characteristic includes blood vessels visible through the skin. Optionally, a liquid is placed between the external body portion and the scanning region, the liquid altering the index of refraction to improve visualization of the at least one characteristic. The first scanning mode may also involve placing a dye and/or a pigment on the external portion to improve viewing of the at least one characteristic.

In the second mode, a transfer member is placed in contact with the external portion of the individual to provide an image on the transfer member, and the image of the transfer member is scanned with the scanner to obtain the scanned image data. Preferably, the image on the transfer member indicates a condition of the external portion. There are many different types of transfer members that could be used. The transfer member could even be part of the image scanner itself. For example, the transfer member could be a window of the scanner that defines the scanner's scanning region.

In one example of the process including the second mode, the transfer member includes adhesive material provided on a backing, the adhesive material of the transfer member being placed in contact with skin (or any other external body portion) and the transfer member being removed from the skin to transfer cells from the skin of the individual to the transfer member. In this example, the amount of cells transferred to the transfer member could be analyzed to diagnose the condition of the dryness of the skin. The adhesive material of the transfer member may be placed in contact with adhesive material of a second transfer member and the transfer members may then be separated to transfer a portion of the skin cells to the second transfer member.

In one other example of the process, the transfer member is placed in contact with an external body portion having a product, such as a cosmetic product, applied thereto, and the image of the scanned image data is representative of at least one characteristic of the product. For example, the external portion could include the lips and the product could be a lip care product or a lip makeup product, such as lipstick.

In an exemplary process where the image of the scanned image data is representative of a product applied to the external body portion, a transfer member in the form of a sheet of material could be placed in contact with lips of the individual and a lip product could be transferred from the lips to the sheet of material. This enables analysis of the non-retention and/or non-transferability characteristics of a makeup product, such as lipstick on the lips.

In another example, the transfer member could be placed in contact with skin, such as facial skin, having foundation makeup applied thereto. Such a process could be used to analyze non-retention and/or non-transferability characteristics of the foundation makeup. For example, when the transfer member is a piece of fabric or an entire article of fabric clothing, such as a blouse, the method could be used to evaluate whether a product causes soiling of clothing and/or whether the product remains on the skin during a period of time.

In a further example, where the transfer member of the second mode includes a moldable material, the moldable material is placed in contact with the skin of the individual to produce, on the moldable material, the surface profile of the skin.

In yet another example, where the transfer member is a hair comb or a hair brush, the comb or brush is passed through hair, and the image on the transfer member includes hair strands and/or skin cells.

Still another example of the second mode involves the use of a transfer member configured to change color in response to a condition of the external portion. For example, the transfer member could be formed of litmus paper.

The first and second scanning modes could be combined. For example, the modes could occur either simultaneously or one after the other.

In one preferred embodiment, a first computer associated with the image scanner is located at a first location, and the process further comprises transferring the scanned image data from the first computer associated with the image scanner to a second computer located at a second location remote from the first location. The transferring may include transmitting the scanned image data via the Internet, or shipping a data storage medium, such as a CD ROM or computer disk, to the second location.

Other information may also be transferred to the second location. For example, questionnaire answers relating to the condition of the external portion and/or the product applied to the external portion may be transferred to the second location. Billing and/or payment information could also be sent to the second location.

In a preferred embodiment, an image is displayed at the first location and/or the second location. The displayed image corresponds to the scanned image data, and includes representations of the characteristics. Analysis of these characteristics may include viewing the displayed image. At least part of the analysis and/or the diagnosis determination may be performed manually or by a computer, such as the second computer.

In another preferred practice of the invention, the process includes sending the scanned image data to a plurality of locations so that the characteristic(s) may be analyzed numerous times.

In another aspect, the process of the present invention may include determining a recommendation of one or more treatments for the diagnosed condition(s) of the external portion, and providing the treatment recommendation so that the external portion of the individual may be treated according to the recommendation. A remotely located computer may at least partially determine the treatment recommendation.

In one aspect of the present invention, the recommendation is a recommendation regarding use of a cosmetic product and/or a dermatological product, such as a makeup product, a care product, a hair product, a skin product, and a sun exposure product. For example, it could be a recommendation regarding application of the product to the external portion.

In the present application, a cosmetic product is a product as defined in the Directive 76/768/EEC as amended by the Council Directive 93/35/EEC of 14 June 1993.

A cosmetic treatment is a non therapeutic treatment with a cosmetic product as defined above.

Optionally, product ordering information is provided along with the recommendation.

The condition diagnosis, product feature diagnosis, and/or the treatment recommendation may be provided to the individual and/or a treatment provider. This information may be sent via the Internet or any other form of communication means.

Another aspect of the process includes monitoring status of the external portion during treatment for the diagnosed condition. For example, the monitoring process may include repeating at least the obtaining of the scanned image data and the analysis of the characteristic(s). A recommendation for an additional treatment could be provided based on the monitored status. In addition, the individual may be provided with information regarding the effectiveness of the treatment.

Another aspect relates to evaluating a product applied to the external portion. Such a process involves the analysis of one or more characteristics of the product, particularly for cosmetic products.

The process may also involve one or more databases. For example, the process may include collecting information relating to the scanned external portion and/or the product to form a database for use in the diagnoses, treatment recommendation determinations, product evaluations, and product formulations. In another example, the analysis of

Another aspect relates to evaluating a product applied to the external portion. Such a process involves the analysis of one or more characteristics of the product, particularly for cosmetic products.

The process may also involve one or more databases. For example, the process may include collecting information relating to the scanned external portion and/or the product to form a database for use in the diagnoses, treatment recommendation determinations, product evaluation, and product formulations. In another example, the analysis of the characteristic may include comparing an image formed from the scanned image data to at least one image formed from image data stored in an image database.

In another aspect, the scanned image data includes data regarding color of the characteristic. This enables the analysis at the second location to include an evaluation of the color of the characteristic(s).

In yet another aspect, the scanner emits light onto the external portion during scanning in the first mode and emits light on the transfer member during scanning in the second mode.

In a further aspect, a calibration member is scanned along with the external portion and/or the image of the transfer member. The calibration member preferably has a predetermined size and/or a predetermined color.

In an even further aspect, the scanned image data includes data relating to multiple scanned images or a single scanned image.

In yet another aspect, the transfer member is treated to enhance the image on the transfer member.

In yet another aspect, the external body portion and/or the transfer member could be analyzed with various types of analysis equipment. In addition, a trained person, such as a clinician, could conduct an analysis of the external portion.

It is to be understood that both the foregoing general description and the following detailed description are exemplary, and are intended to provide further explanation of the invention as claimed.

### BRIEF DESCRIPTION OF THE DRAWINGS

The accompanying drawings are included to provide a further understanding of the invention and are incorporated in and constitute a part of this specification. The drawings illustrate various aspects of embodiments of the process according to the invention and, together with the description, serve to explain the principles of the invention. In the drawings,
Fig. 1 is a schematic view of an example of system capable of being used to practice the process of the present invention;
Fig. 2 shows a schematic view of scanned image data being obtained in a first mode by directly scanning an external portion of the body with an image scanner show in Fig. 1;
Fig. 3 is a plan view of an adhesive transfer member configured to be used to obtain scanned image data in a second mode;
Fig. 4 is a schematic view showing the adhesive transfer member of Fig. 3 being placed in contact with facial skin of an individual;
Fig. 5 is a plan view of the adhesive transfer member of Fig. 4 showing skin cells transferred to the transfer member after removal of the transfer member from the skin;
Fig. 6 is a perspective view of scanned image data being obtained in the second mode by scanning the transfer member of Fig. 5;
Fig. 7 is a view of a scanned image showing dry skin from a leg, wherein data for the image was obtained according to the first mode of Fig. 2;
Fig. 8a is a view of a scanned image showing pigment spots on skin, wherein data for the image was obtained according to the first mode of Fig. 2;
Fig. 8b is a view of a scanned image similar to that of Fig. 8a, wherein contact oil has been placed on the spot prior to scanning;
Fig. 9 is a view of a scanned image of root portions of two strands of hair, wherein data for the image was obtained according to the first mode of Fig. 2;
Fig. 10 is view of a scanned image of an entire strand of hair, wherein data for the image was obtained according to the first mode of Fig. 2;
Fig. 11 is a view of a scanned image of a nail clipping of a fingernail, wherein data for the image was obtained according to the first mode of Fig. 2;
Fig. 12 is a view of a scanned image of a nail showing bed capillaries, wherein a liquid is used to modify the index of refraction and wherein data for the image was obtained according to the first mode of Fig. 2;
Fig. 13 is a view of a scanned image of a finger tip showing a fingernail, wherein data for the image was obtained according to the first mode of Fig. 2;
Fig. 14 is a view of a scanned image of skin cells transferred to a transfer member, wherein data for the scanned image was obtained in the manner shown in Fig. 6;
Fig. 15 is a view of a scanned image of top, front teeth, wherein data for the scanned image was obtained in a manner similar to that shown in Fig. 2;
Fig. 16 is a view of a scanned image of a tissue paper transfer member including a lipstick imprint of lips, wherein data for the scanned image was obtained in a manner similar to that shown in Fig. 6;
Fig. 17 is a view of a scanned image of skin including pigmentation and micro cuts, wherein data for the scanned image was obtained in a manner similar to that shown in Fig. 2;
Fig. 18 is a view similar to that of Fig. 17 showing skin from another external portion of the body, wherein data for the scanned image was obtained in a manner similar to that shown in Fig. 2;
Fig. 19 is a view of a scanned image showing dry skin, wherein data for the scanned image was obtained in a manner similar to that shown in Fig. 2; and
Fig. 20 is a view of a scanned image of skin of the cheek showing small micro vessels and facial hair, wherein data for the scanned image was obtained in a manner similar to that shown in Fig. 2.

### DETAILED DESCRIPTION OF EMBODIMENTS

Reference will now be made in detail to embodiments of the invention, examples of which are illustrated in the accompanying drawings. Wherever possible, the same reference symbols are used in the drawings and the description to refer to the same or like parts.

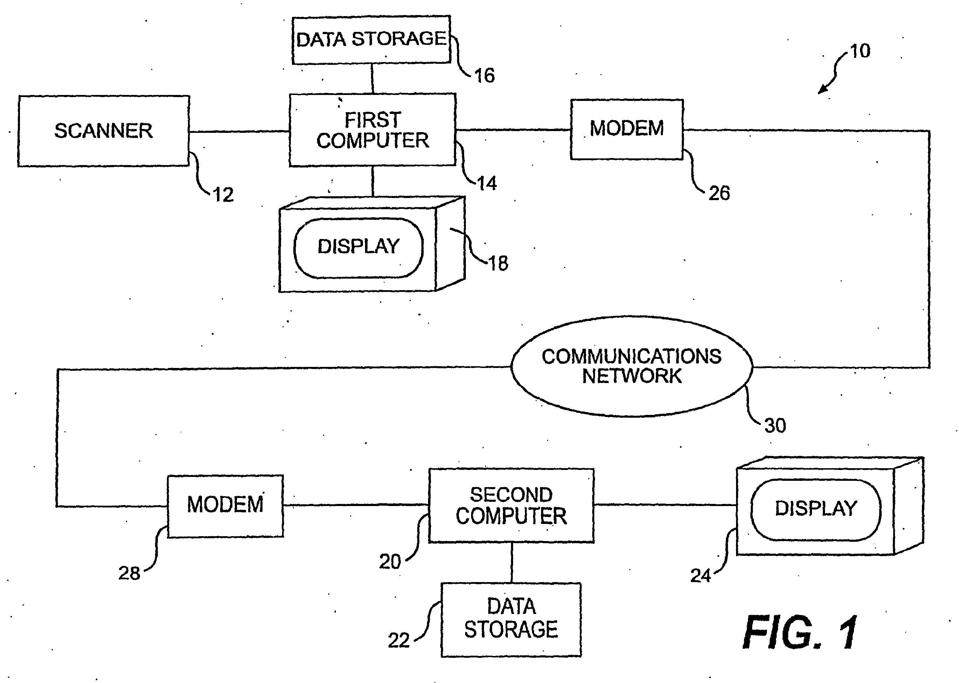

Fig. 1 shows an example of a system 10 that could be used to practice a process according to the present invention. The system 10 includes an optical image scanner 12, a first computer 14 associated (via any type of communication link, including a phone line) with the scanner 12, a data storage 16 for the first computer 14, and a visual display screen 18 for the first computer 14. The system 10 also includes a second computer 20 linked to a data storage 22 and a visual display screen 24. Preferably, the optical image scanner 12 and first computer 14 are provided at a first location remote from a second location where the second computer 20 is located. Respective modems 26 and 28 are provided to link communication between the computers 14 and 20 via a communication network 30, such as the Internet.

The first and second computers 14 and 20 could be configured in many different ways. In one implementation the computers 14 and 20 are conventional personal computers typically found in home or office environments. Many other types of devices, including those that are hand-held, may also be used as long as they are capable of processing scanned image data generated by an image scanner.

One of the initial stages of the process according to the present invention involves obtaining scanned image data with the optical image scanner 12. Preferably, the optical image scanner 12 is a conventional, optical, image scanner typically used to scan documents and/or photographs in a home or office environment. Many different types of commercially available image scanners could be used in the practice of the present invention. For example, the scanner could be a flat bed scanner, a hand-held scanner, a slide scanner, or even a combined scanner and facsimile device. Preferred scanners have a resolution high enough to produce a 2-dimensional scanned image showing viewable details that are normally taken into account during analysis of the condition of an external portion of an individual. For example, the image scanner 12 could have a resolution of up to about 4800 dots per inch (dpi).

Scanners for use in the process of the present invention preferably emit light on an object being scanned. The object being scanned may absorb part of this light, reflect part of it, and/or permit passage of part of it through the object. The scanner preferably detects the reflected portion of light. The emitting of light during scanning enables the scanned image to be relatively standardized and relatively unaffected by ambient light conditions because preferably all, or a substantial portion, of the light detected by the scanner originates from the scanner.

Preferably, the scanner 12 includes one or more light-emitting scanning elements that are moved relative to the object being scanned. Alternatively, the scanner 12 could be configured such that the object being scanned is moved relative to the light-emitting scanning elements. Rather than providing an instantaneous scan of an entire object being scanned, the scanner 12 is preferably configured to sequentially scan different portions of an object in either a block-by-block, line-by-line, or point-by-point manner, for example.

The preferred scanner may have a relatively short depth of field for its scanning (i.e., the scanner and the object being scanned are preferably located at a close, predetermined distance to one another during scanning). In one preferred embodiment, the object being scanned is placed in contact with a support during scanning. For example, the support could be part of the scanner, such as a window defining a scanning region, or the support could be separate from the scanner.

The preferred scanner is also preferably a color scanner configured to produce scanned image data including color data. A color scanner is preferred because it enables a skin diagnosis, for example, that takes into account color. One possible scanner, used to produce the scanned images shown in the drawings, is an EPSON Perfection, model 1200 Photo scanner having a maximum resolution of 1200 dpi. Another type of possible scanner is a QUBYX Lynx A3 scanner having a resolution of between 2400 and 4800 dpi.

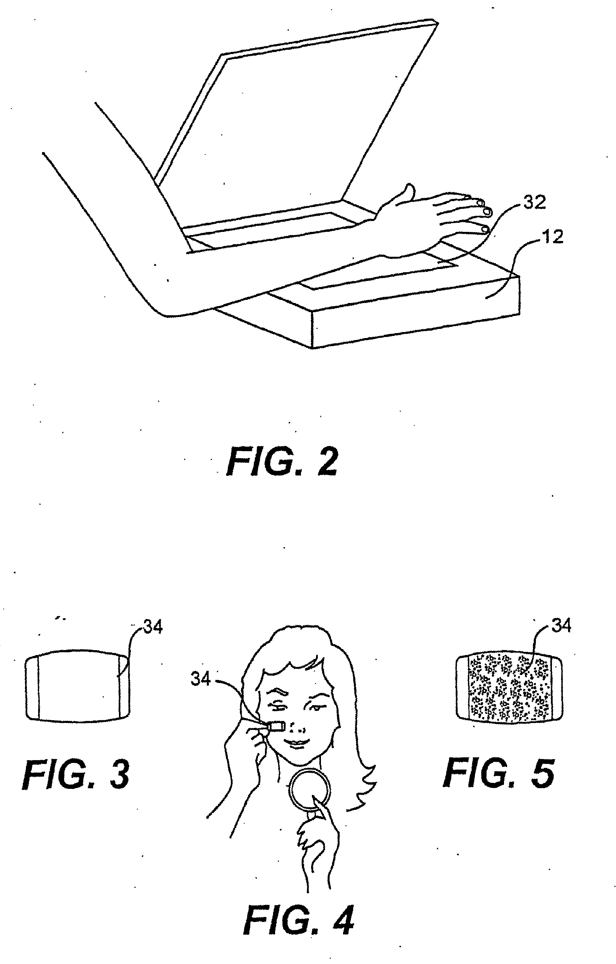

Preferably, the scanned image data is obtained with the scanner 12 by practicing either a first mode, a second mode, or some combination of the first and second modes. Fig. 2 shows an example of the first scanning mode. In the first mode, the external portion of an individual (i.e., the arm shown in Fig. 2) is placed in the vicinity of a scanning region 32 of the scanner, and the external portion is scanned with the image scanner 12 to obtain the scanned image data. In the example shown in Fig. 2, the scanner 12 includes a scanning region 32 configured in the form of a glass window pane that makes contact with an object being scanned, the external portion of the individual is preferably placed into contact with this scanning region 32 during the scanning. Preferably, the scanner shown in Fig. 2 is a flat bed scanner, and the external portion of the individual is moved into contact with the glass window pane of the scanner 12. If, on the other hand, the scanner is a hand-held scanner (not shown), the scanner can be moved to place its scanning region into contact with the external portion of the individual.

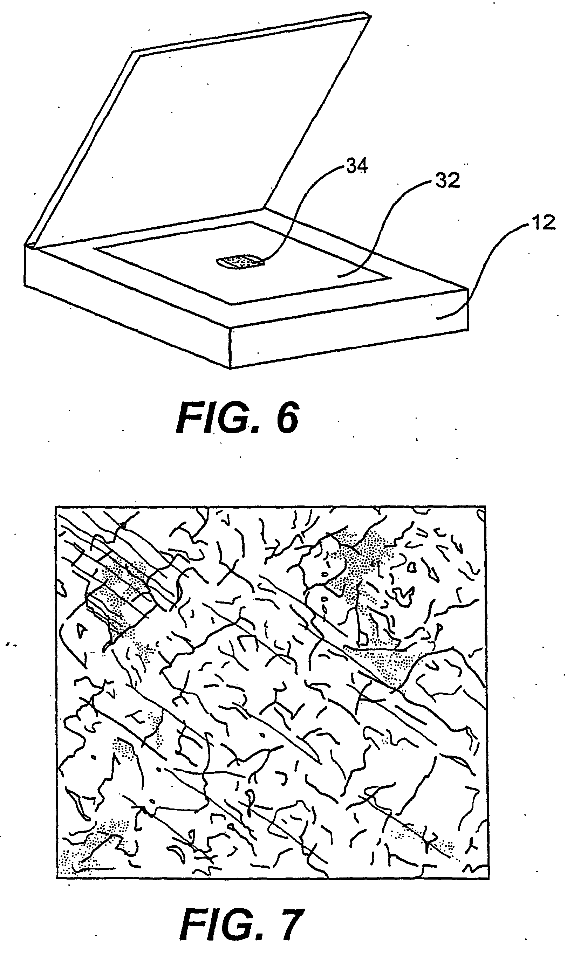

The first scanning mode could be used for the diagnosis of a skin condition. For example, when diagnosing a skin condition, such as dry skin, the skin of an individual's face, arm, leg, hand, foot, or torso could be brought in the vicinity of (i.e., placed near or against) the scanning region 32 of the scanner 12 during scanning. Fig. 7 shows an example of a scanned image showing dry skin from a leg, wherein the image was scanned while the scanning region 32 was in contact with the skin. The first mode scanning might also be used in the diagnosis of many other skin conditions, such as psoriasis, vitiligo, or melanoma, for example

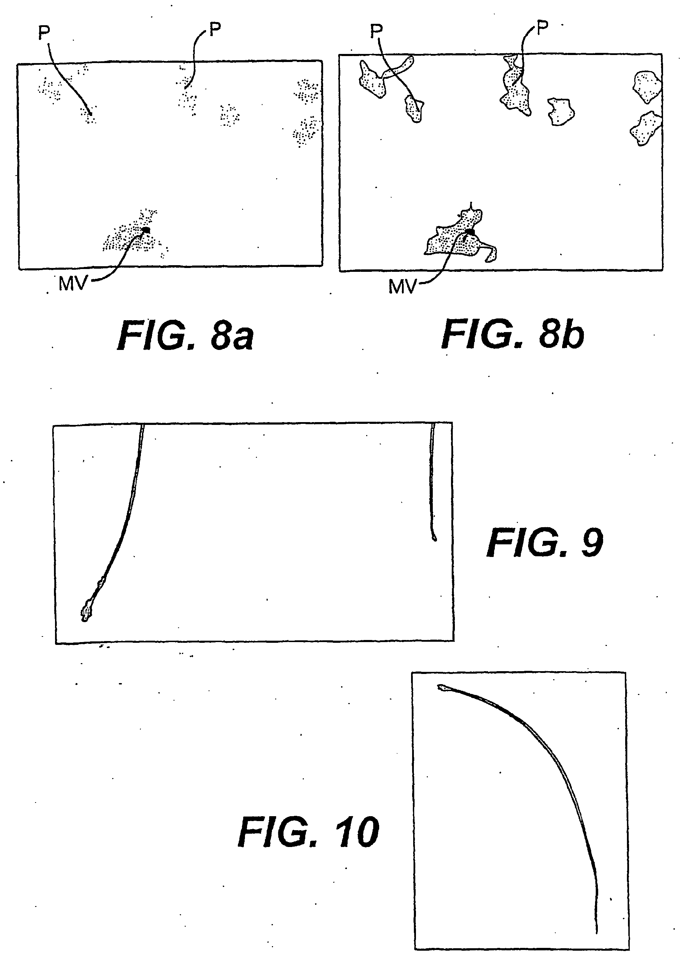

Scanning in the first mode could also be used to diagnose certain pigmented areas on the skin and/or blood vessels, such as micro vessels, visible through the skin. Fig. 8a shows an example of a first mode scanned image showing a skin region containing pigment spots P and a visible micro vessel MV. Fig. 8b is an example of a scanned image showing the skin region of Fig. 8a wherein a liquid (i.e., contact oil) has been placed on the spot prior to scanning in order to alter the index of refraction and thereby improve viewing of the skin characteristics, such as the pigment spots P and the micro vessel MV. This aspect of the process may be used to diagnose the condition of blood vessels visible through the skin and to detect acrosyndromes or couperosis, for example.

To further enhance viewing, a dye and/or pigment (i.e., a fluorescent pigment) could be placed on the skin prior to the scanning.

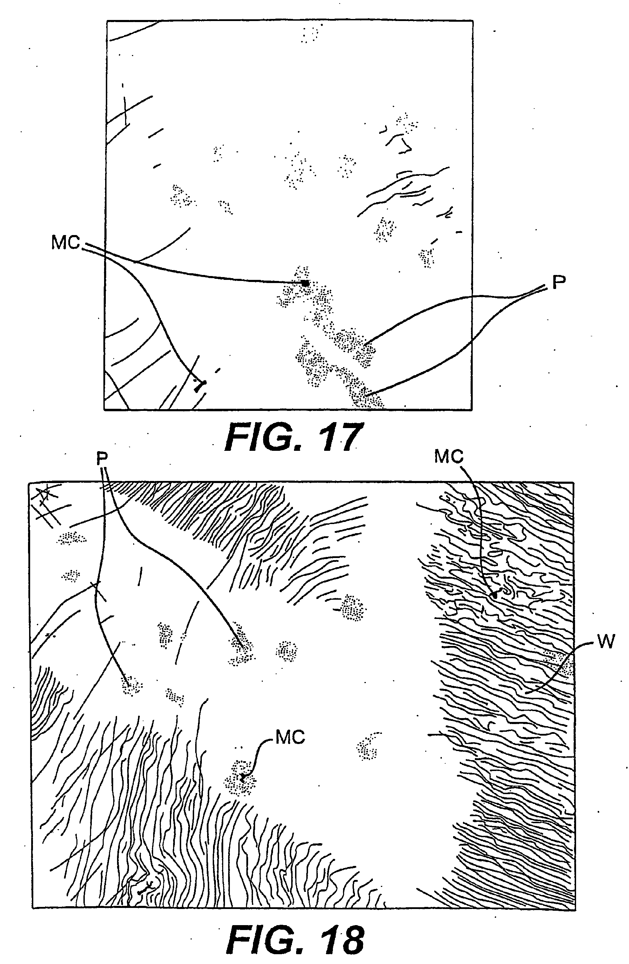

Fig. 17 is an example of another first mode scanned image showing a skin region including pigment spots P and micro-cuts MC caused, for example, by shaving. Fig. 18 is another example of a first mode scanned image showing a skin region similar to that of Fig. 17 and also including wrinkles W.

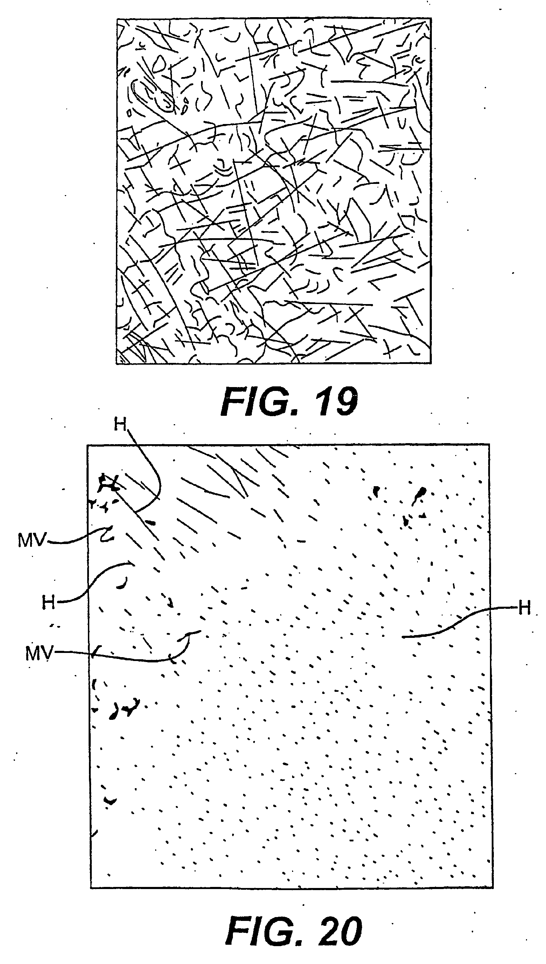

Fig. 19 is an example of a first mode scanned image showing a skin region having cracks indicating a significant number of dry and/or dead skin cells. Fig. 20 shows another example of a first mode scanned image of a skin region from an area such as the cheek, wherein the skin region includes micro vessels MV an a number of facial hair strands H, some of which have been shaved shorter than others.

In addition to being used in analysis of skin, the first mode could also be used to scan the image of a strand of hair for use in the diagnosis of certain hair conditions, such as determining the thickness or length of a strand of hair or the status of a hair root. For example, the strand could be either a strand of hair from the scalp of the individual, an eyelash of the individual, or an eyebrow hair of the individual. Fig. 9 shows an example of a scanned image of the root portions of two separate strands of hair. Fig. 10 shows an example of a scanned image of an entire strand of hair. Each of the images of Figs. 9 and 10 was scanned while the hair strand was placed against the scanning region 32.

The hair strands shown in Figs. 9 and 10 could be obtained in a variety of different ways. For example, the hair strands could be pulled from the skin of the individual, removed during brushing or combining, or collected from clothing or a drain of a shower or bath.

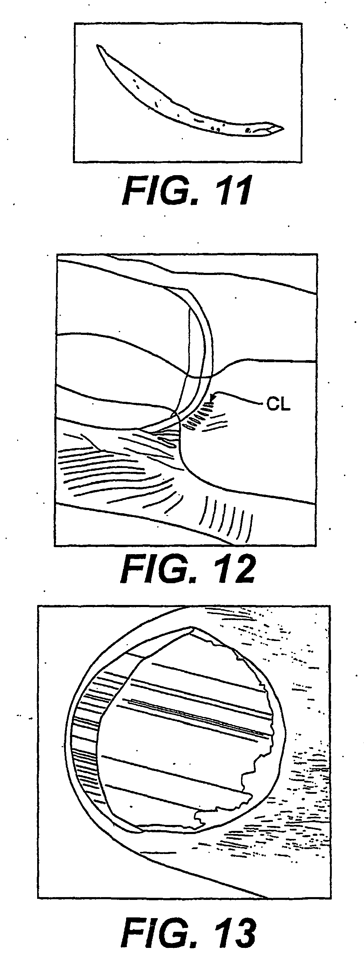

The first scanning mode could also be used to scan an image of a fingernail or a toenail for use in diagnosis relating to pathology, ungual state, onychomycosis, or split nails, for example. Fig. 11 shows an example of a scanned image of a nail clipping of the fingernail, wherein the image was obtained by scanning when the nail clipping was in contact with the scanning region 32. A scanned image like that of Fig. 11 may be used in the diagnosis of nail delamination. Fig. 13 shows an example of a scanned image showing a fingernail and cuticle, wherein the image was obtained by scanning when the finger tip was in contact with the scanning region of the scanner. Fig. 12 shows a scanned image similar to Fig. 13, wherein a liquid (i.e., oil) was placed on the finger prior to scanning to improve visualization of capillary loops CL near the cuticle of the finger. Such an image could be used for the diagnosis of acrosyndromes, such as Raynaud's syndrome.

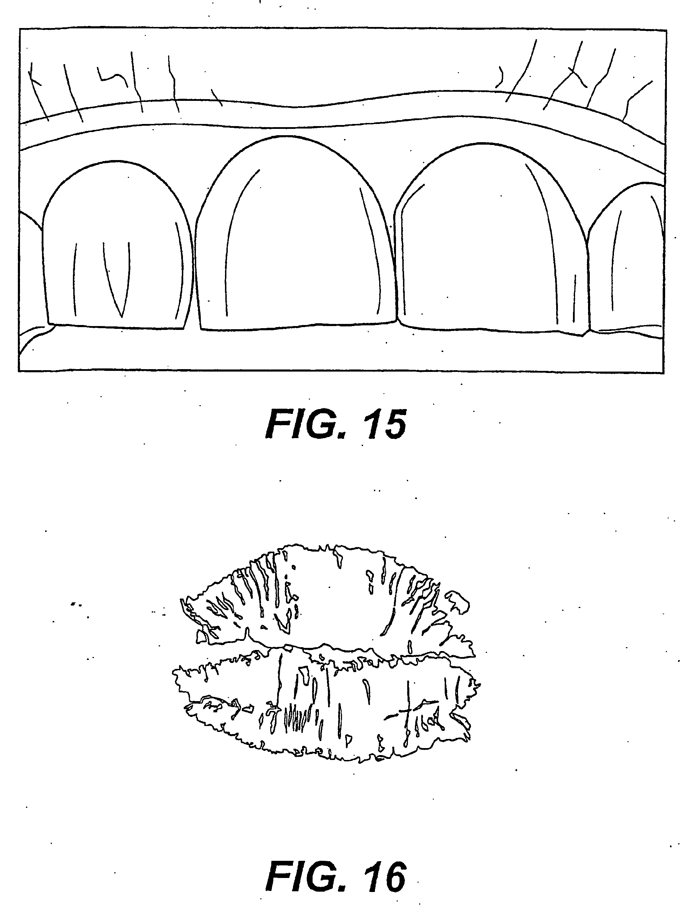

The first mode scanning could also be used to scan other exterior portions of the body. For example, Fig 15 shows an example of a scanned image of top front teeth. Such an arrangement could be used to diagnose a number of different conditions of the teeth.

As mentioned above, the scanned image data could also be obtained in a second mode different from the first mode. The second mode includes placing a transfer member in contact with the external portion of the individual to provide a visual image on the transfer member, and then scanning the visual image of the transfer member with the scanner to obtain the scanned image data. There are many different types of transfer members that could be used for the second mode. For example, the transfer member could include either adhesive material provided on a backing, a sheet of absorbent material, a piece of fabric, an article of fabric clothing (i.e., a blouse), a piece of moldable material, a hair brush or comb, or even the window defining the scanning region 32 of the scanner 12.

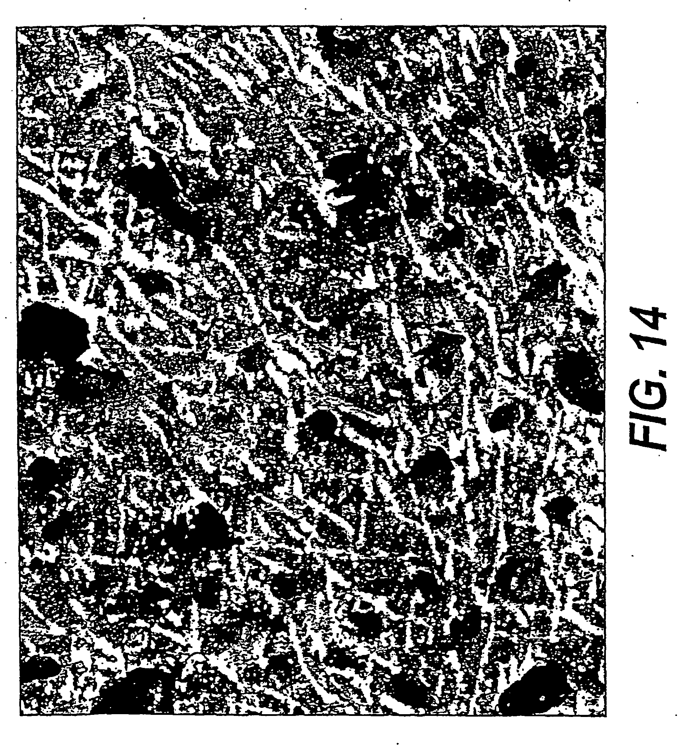

Fig. 3 shows an example of a transfer member 34 including adhesive material provided on a backing. In one preferred embodiment of the invention, the transfer member 34 is a commercially available product called SEBUTAPE. Many other types of alternative configurations are also possible. For example, this type of transfer member could simply be a piece of relatively transparent plastic tape, such as SCOTCH tape manufactured by 3M. As shown in Fig. 4, the adhesive material of the transfer member 34 is placed in contact with skin (i.e., of the face) and, as shown in Fig. 5, when the transfer member is removed from the skin, skin cells and possibly also sebum are transferred from the skin of the individual to the transfer member. As shown in Fig. 6, the transfer member 34 is then scanned with the scanner 12 (for example, by placing it in contact with the scanning region 32) to obtain a scanned image showing the transferred skin cells and/or sebum. An example of this type of scanned image is shown in Fig. 14, wherein open areas between aggregates of skin cells show cohesion between the skin cells, separation of skin cells, and valleys in the skin. With such an arrangement, the amount of cells transferred to the transfer member could be analyzed to diagnose the condition of the dryness of the skin. In addition, this could be used to diagnose desquamation.

When the removal of the transfer member 34 from the skin results in a significant amount of skin cells and/or sebum being transferred to the transfer member 34, the amount may be reduced by placing the adhesive side of the transfer member 34 in contact with the adhesive of another transfer member and then separating the two transfer members to transfer amounts of the skin cells and/or sebum to both transfer members. Such a procedure could be used in order to diagnose the size of individual cells where an overabundance of cells on the transfer member make the analysis difficult.

In an example of the process, a transfer member may placed in contact with an external body portion having a product, such as a cosmetic product, applied thereto, so that a transfer image relating to one or more characteristics of the product is created on the transfer member. For example, the external portion could include the lips and the product could be a lip care product or a lip makeup product, such as lipstick. One possible type of transfer member is a sheet of absorbent material and this sheet could be in the form of a paper sheet, such as a facial tissue, toilet tissue, or paper towel. The sheet of material could be placed in contact with lips of an individual to transfer a lip product, such as lipstick, from the lips to the sheet of material. Fig. 16 shows an example of a scanned image of tissue paper including an imprint of lips formed, for example, from lipstick. This type of scanned image could be used to diagnose the non-retention and/or non-transferability characteristics of lipstick over time. In other words, the process could be used to determine the ability of the lipstick to remain on the lips as a function of time and/or as a function of the number of events when the lips come in contact with other things, such as by kissing. Additionally, such a process could be used to determine coverage of the product on the external body portion.

In another example, a transfer member in the form of a piece of fabric or an article of fabric clothing (i.e., a blouse) could be placed in contact with skin, such as facial skin, having foundation makeup applied thereto. The amount of any foundation makeup transferred to the transfer member could then be scanned with the scanner 12. Such a process could be used to analyze non-retention and/or non-transferability characteristics of the foundation makeup. In particular, the method could be used to evaluate whether a product causes soiling of clothing and/or whether the product remains on the skin during a period of time.

In a further example, the transfer member of the second mode includes a moldable material, such as modeling clay or a malleable paste. The moldable material could be pressed against the surface of the skin to produce the surface profile of the skin on the moldable material. The moldable material could then be scanned to produce a scanned image. Such a scanned image could be used for the analysis of micro-reliefs in the skin.

In still another example, the transfer member could be the window of the scanning region 32. In such an arrangement, a visible image would be created on the window after contact of an external body portion with the window, and removal of the body portion prior to scanning. For instance, a lip imprint like that of Fig. 16 could be placed on the window, for example with lipstick. This could be used in the analysis of the non-transferability of a lip product.

The process of the present invention could be practiced to determine both the coverage and non-transferability of a product applied to the external body portion. For example, after applying a cosmetic product to a skin portion, the skin portion could be placed in contact with the scanning region 32 during scanning to obtain image data for an image representing coverage (i.e., homogeneity) of the product on the skin portion. After removing the skin portion from the scanning region 32, any of the product transferred from the skin portion to the scanning region 32 (which is also the transfer member in this example) could then be scanned to obtain scanned image data for an image relating to the non-transferability of the product.

A hair brush or a hair comb could also provide a transfer member. With this type of an arrangement, the brush or comb would be passed through the hair to collect hair strands and/or skin cells and then the brush or comb would be scanned in a manner like that of Fig. 6. This could be used to diagnose the extent of hair loss or dandruff, for example.

In an alternative process according to the invention, the transfer member may be configured to change color when the transfer member is placed in contact with the external body portion and the color change may provide an indication of the condition of the external body portion. For example, the transfer member could be configured in the form of litmus paper capable of measuring PH of the skin by changing color.

Optionally, the transfer member and/or the external body portion could be treated before the transfer member is placed on the external body portion. Such treatment might enhance gathering of material on the transfer member and/or viewing of features on the transfer member.

The first and second scanning modes could be combined in a number of different ways to obtain scanned image data relating to an exterior portion of a body. For example, both a transfer member and an external body portion could be placed in contact with the scanning region 32 and then scanned substantially simultaneously. Alternatively, scanning in the first and second modes may occur one after the other so that scanned image data from both of these modes may be used.

In one example of a process combining multiple modes and analysis of both external portion characteristic and product characteristics, skin of the cheek could be placed in contact with the scanning region 32 during scanning to obtain image data relating to an image representative of normal transparency of the skin. Then, a hydrating cream that improves skin transparency could be applied to the cheek and cheek could be again scanned while in direct contact with the scanning region 32 to obtain scanned image data relating to the improved visibility provided by the cream. After the cheek is finally removed from the scanning region 32, any cream transferred to the scanning region 32 (i.e., the scanner window provides the transfer member) could then be scanned to obtain scanned image data relating to non-transferability of the cream.

Optionally, a calibration member may be scanned along with the external body portion and/or transfer member. For example, the calibration member could have a predetermined size and or color that would enable calibration of an image formed from the scanned image data (for example, via image processing software such as Photoshop) to provide a more exact indication of the size and/or color of characteristics.

When the scanned image data has been obtained, the process according to the present invention further includes analyzing one or more characteristics of the external body portion and/or the product applied to the external portion, and determining a diagnosis of one or more conditions of the external portion and/or one or more features of the product. There are many different ways in which this analysis and determination may take place. For example, the analysis could include a person viewing an image (displayed on the first or second display screen 18, 24 for example) formed from the scanned image data obtained with the image scanner 12, and making a determination of a diagnosis based on this viewing. Alternatively, a computer program running on the first or second computer 14, 20 could perform at least a portion of the analysis and diagnosis. The person and/or computer performing the analysis and/or diagnosis could provide a grade indicating the condition of the external portion and/or product performance, and this grade could be stored in one of the data storages16, 22.

The analysis according to the present invention could combine both an analysis of one or more images formed from the scanned image data and any other type of analysis for external body portions and/or cosmetic products. For example, the other analysis could be an analysis using conventional analysis equipment used for analyzing external body portions and/or cosmetic products applied to external body portions. In particular, the process could include usage of equipment typically used by specialists during examinations. For example, the process of the present invention could include the use of corneometer, a dermal torque meter, an image analyzer, a sebumeter, a PH meter, or a device for measuring hydration of the skin. The other analysis could also be an analysis performed by a trained person, such as a clinician, directly viewing the external portion, for example, at a location where products are sold. The additional analysis could be used to confirm the results of the analysis via the scanned image data. During the course of this analysis, a grade representative of the condition of the external portion and/or performance of the product could be provided. This grade could then be stored, for example, in one of the data storages 16, 22.

In one preferred practice of the present invention, the scanned image data is transferred from the first computer 14 to the second computer 20 via the communications network 30. Alternatively, the data could be stored on a data storage medium, such as a computer disk, CD, or other information storage means, and this data storage medium could be shipped to the location of the second computer 20. In addition, the scanned image data could be stored in the first and/or second data storages 16, 22.

Optionally, the scanned image data could be transferred to the second computer 20 along with written information, such as answers to a brief questionnaire regarding the condition of the exterior body portion and/or product applied to the external portion. These questionnaire answers may then be considered in conducting the analysis and diagnosis. In addition, billing information and/or payment information may also be sent along with the scanned image data.

Preferably, the second computer 20 is located at a diagnosis area where an image can be created from the scanned image data transferred from the first computer 14. Optionally, this image could be displayed at the second location on the second display screen 24. The image preferably contains representations of one or more of the characteristics of the external body portion and/or the product applied to the external portion. These characteristics are analyzed at the second location to provide a diagnosis of one or more conditions of the external portion and/or one or more features of the product. At least part of the analysis could involve a person viewing a displayed image at the second location. In addition, some or at least substantially all of the analysis could be performed automatically by the second computer 20. For example, the image could be analyzed at least partially by means of an image analysis software operating on the second computer 20.

Optionally, the first computer 14 and/or the second computer 20 could modify the scanned image data to improve the viewing of certain characteristic of the external body portion and/or the product. For example, image modification software, such as Photoshop, could be used to enhance viewing of the characteristics shown in the images. Such software could be used to digitally magnify portions of images being displayed to facilitate analysis and diagnosis.

Preferably, the results of the diagnosis are provided to the individual and/or a treatment provider for the individual. For example, the diagnosis could be sent via the communications network 30.

When one or more conditions of an individual's external portion have been diagnosed, a recommendation for treatment of the condition(s) may be determined. Preferably, this recommendation is provided to the individual and/or a treatment provider so that the external portion of the individual may be treated according to the recommendation.

The recommendation could be determined at least in part by a manual process or an automated process. For example, the recommendation could be determined by selecting, from one of the data storages 16 and 22, treatments based on the diagnosed condition. The recommendation could be provided to the individual and/or treatment provider by sending it via the communications network 30. In addition, information relating to the diagnosis could also be provided along with the recommendation.

In one aspect of the present invention, the recommendation is a recommendation regarding use of at least one of a cosmetic product and a dermatological product. A variety of products could be recommended using this technique. For example, the recommended products may be chosen from makeup products, care products (both therapeutic and non-therapeutic), hair products, skin products, and sun exposure products (i.e., sun screen or after-sun products). The recommendation could be a prescription for a particular product.

The treatment recommendation may include a recommendation regarding application of a product to the external portion. Optionally, product ordering information may be provided along with the recommendation.

In certain circumstances, the treatment recommendation might not involve usage of a particular product. For example, the treatment recommendation could be advice regarding hygiene or cleaning for a body portion.

The scanned image information transferred from the first computer 14 to the second computer 20 could also be used for monitoring the status of the condition of the external portion during treatment. For example, skin pigmentations could be monitored over time to determine effectiveness of a treatment; or sizes of skin cells could be monitored over time to determine skin cell renewal rate. Optionally, an additional recommendation for a treatment could be provided based on the monitored status. Such a recommendation could be a recommendation regarding application of at least one cosmetic product and dermatological product to the external portion, and product ordering information can be provided along with the recommendation. In addition, the process could involve providing the individual with information regarding the effectiveness of the recommended treatment. The monitoring could include repeating the obtaining of the scanned image data and the analysis. Each monitoring could include providing a grade representative of the condition of the external portion and/or product performance.

One more additional aspect involves collecting information relating to the scanned external portion to form a database for use in at least one of further diagnoses, further recommendation determinations, further product evaluations, and/or product formulations. For example, a neural network could be established that would add information to its database and establish some form of artificial intelligence system. Such a database could be used when conducting further analysis of characteristics of external body portions and/or products. For example, an image formed from the scanned information could be compared to an image formed from a database stored in one of the data storages 16 and 22. The database could also be used to evaluate different product formulations to select an appropriate formulation. Optionally, the database could include information relating to one or more grades representative of the condition of the external portion and/or product performance.

Another possible practice of the present invention involves sending the scanned image data to a plurality of different locations, for example via the communications network 30, to permit substantially simultaneous analysis at a plurality of different areas. For example, such a practice of the present invention could permit a team of experts in different areas to diagnose external body conditions and/or evaluate products somewhat simultaneously.

The process of the present invention could be practiced to diagnose many different types of condition. For example, the process could be practice to diagnose skin conditions, such as elasticity, dryness, cellulitis, sweating, aging, wrinkles, melanoma, exfoliation, desquamation, homogeneity of color, micro-circulation, shininess, softness, smoothness, matitty, hydration, sebum production, cleanliness, irritation, redness, vasomotion, vasodilation, vasoconstriction, pigmentation (including freckles), PH, whitening, dying or coloring, insect bites, growths, lesions, wounds, post surgical incisions, etc., for example.

With regard to hair, the process may be practiced to diagnose dying, curling, scales, keratin plugs, length, dryness, oiliness, dandruff, lice or other parasites, thickness, density, root conditions, split ends, hair loss, staging, etc., for example.

For fingernails or toenails, the process could be practiced to diagnose lines, spots, thickness, skin at the base of the nail, delamination, curvature, brilliancy, length, psoriasis, etc., for example. In addition, diagnoses relating to the teeth may include color, enamel coverage, surface smoothness, whiteness, etc., for example.

When the process involves a treatment recommendation for the external portion, there are a variety of different treatment recommendations that could be provided. For example, treatment recommendations for skin conditions could include use of nourishing cream, anti-wrinkle cream, moisturizer, or keratinous cream; applying a solution of salicylic acid; or removal of dead skin cells via exfoliation, etc., for example. Possible hair treatment recommendations may include use of special shampoos or other products for treating hair loss, split ends, dandruff; or types of hair trimming, etc., for example. For nails, possible treatment recommendations include, pushing of the cuticles, applying cuticle cream, softening of the cuticles, polishing nails, use of nail varnish, application of nail care creams (i.e., for treating psoriasis), etc. for example. Regarding the teeth, possible treatment recommendations relate to brushing, flossing, and use of whiteners, tart removers, or nicotine removers, etc., for example.

The process according to the present invention could preferably have a number of different advantages. For example, the process preferably could obtain an image with a very high resolution as long as the scanner has that capability. Commercially available document scanners have resolutions up to 4800 dpi, for example. Such high resolutions are greater than those of conventional photographs, and not obtainable with a simple direct viewing of an external portion through a magnifying glass.

The invention could preferably be practiced with equipment typically available to most Internet users.

When a color scanner is used, the color image allows for very accurate diagnosis of color related conditions.

The use of a scanner is advantageous because lighting can be automatically standardized with this type of digitizer. The external portion or transfer member can be scanned by directly placing it in contact with the scanning region, for example, and directly acquiring, point by point, colorimetric coordinates of the image. This is not the case with pictures (either film-based or digital) produced in a home or professional setting.

One other relatively significant advantage relates to the fact that the images are directly transferable to a cosmetician or dermatologist electronically, preferably without any manipulation.

Another advantage relates to the ability to create the image without regard to the level of external lighting.

A further advantage relates to the ability to monitor the change in pathology or effectiveness of a treatment without having to travel.

Of course, many aspects of the invention could be practiced without necessarily accomplishing one or more of these advantages.

It will be apparent to those skilled in the art that various modifications and variations can be made to the structure and methodology of the present invention without departing from the scope or spirit of the invention. In view of the foregoing, it is intended that the present invention cover modifications and variations of this invention, provided they fall within the scope of the following claims and their equivalents.

## Claims

1. A process for diagnosing, based on scanned information, one or more conditions of an external body portion and/or one or more features of at least one product applied to the external body portion, the process comprising:
obtaining, with an optical image scanner (12) configured in the form of a scanner for scanning documents, scanned image data relating to at least one characteristic of
a non-dermatoglyphic external portion of an individual, and/or
at least one product applied to the external portion;
analyzing, based on the image data, the at least one characteristic; and
determining a non medical diagnosis of
at least one condition of the external portion, and/or
at least one feature of the product applied to the external portion.

2. The process of claim 1, wherein the characteristic of the external portion includes at least one of wrinkles, crows eyes, blood vessel networks visible through the skin, skin pores, cosmetic materials applied to an external body portion, viewable features of hair strands including roots, viewable features of skin including pigmentations and groups of skin cells, viewable features of fingernails and toe nails, and exteriorly viewable features of teeth.

3. The process of claim 1, wherein the characteristic of the product includes at least one of non-transferability, product coverage, brilliancy, coloring, greasiness, interactions between the skin and the product, product thickness, and product amount

4. The process of claim 1, wherein the obtaining of the scanned image data includes placing the external portion of the individual in the vicinity of a scanning region of the scanner, and scanning the external portion with the image scanner to obtain the scanned image data.

5. The process of claim 4, wherein the external portion of the individual is placed into contact with the scanning region of the scanner.

6. The process of claim 5, wherein the scanner is a flat bed scanner and wherein the external portion of the individual is moved into contact with the scanning region

7. The process of claim 5, wherein the scanner is a hand-held scanner and wherein the scanner is moved into contact with the external portion of the individual.

8. The process of claim 4, further comprising placing liquid between the external body portion and the scanning region, the liquid altering the index of refraction to improve viewing of the at least one characteristic.

9. The process of claim 4, further comprising placing at least one of a dye and a pigment on the external portion to improve viewing of the at least one characteristic.

10. The process of claim 4, wherein the obtaining of the scanned image data further includes placing a transfer member in contact with the external portion of the individual to provide an image on the transfer member, and wherein the scanning further includes scanning the image of the transfer member with the scanner.

11. The process of claim 10, wherein the scanning of the external portion and the scanning of the image of the transfer member occur one of simultaneously and one after another.

12. The process of claim 1, wherein the obtaining of the scanned image data includes placing a transfer member in contact with the external portion of the individual to provide an image on the transfer member, and scanning the image of the transfer member with the scanner to obtain the scanned image data.

13. The process of claim 12, wherein the transfer member includes adhesive material provided on a backing, the adhesive material of the transfer member being placed in contact with skin and the transfer member being removed from the skin to transfer cells from the skin of the individual to the transfer member.

14. The process of claim 13, wherein the analyzing includes evaluating the amount of cells transferred to the transfer member and wherein the diagnosed condition is the dryness of the skin.

15. The process of claim 13, further comprising placing the adhesive material of the transfer member in contact with adhesive material of a second transfer member and separating the transfer members to transfer a portion of the skin cells to the second transfer member.

16. The process of claim 12, wherein the transfer member is placed in contact with an external body portion including a cosmetic product applied thereto, and wherein an image formed from the scanned image data is representative of at least one characteristic of the cosmetic product.

17. The process of claim 16, wherein the external portion includes lips and wherein the cosmetic product is one of a lip care product and a lip makeup product.

18. The process of claim 16, wherein the external portion includes skin and wherein the cosmetic product is foundation makeup.

19. The process of claim 18, wherein the transfer member is formed of fabric.

20. The process of claim 19, wherein the transfer member is an article of clothing.

21. The process of claim 12, wherein the transfer member is a sheet of material, and wherein the process includes placing the sheet of material in contact with lips of the individual and transferring a lip product from the lips to the sheet of material.

22. The process of claim 21, wherein the lip product is lipstick, the diagnosed feature being the non-retention of lipstick on the lips.

23. The process of claim 12, wherein the transfer member is a moldable material, and wherein the process includes placing the moldable material in contact with the skin of the individual to produce, on the moldable material, the surface profile of the skin.

24. The process of claim 12, wherein the transfer member is a window, the window being a portion of the image scanner defining a scanning region.

25. The process of claim 12, wherein the transfer member is one of a hair comb and a hair brush, wherein the placing of the transfer member in contact with the external body portion includes passing said one of the hair comb and the hair brush through hair, and wherein the image on the transfer member includes at least one of strands of hair and skin cells.

26. The process of claim 12, wherein the image on the transfer member indicates a condition of the external portion.

27. The process of claim 12, wherein the transfer member is configured to change color in response to a condition of the external portion.

28. The process of claim 1, wherein the external portion includes at least one of the skin of the individual, at least one strand of hair of the individual, at least one fingernail of the individual, at least one toe nail of the individual, and at least one tooth of the individual.

29. The process of claim 28, wherein the external portion includes the skin of the individual, and wherein the external portion is located on one of the hand, foot, arm, leg, torso, and face of the individual.

30. The process of claim 29, wherein the external portion is located on the lips of the individual.

31. The process of claim 28, wherein the external portion includes said at least one strand of hair, and wherein said at least one strand of hair is one of a strand of hair from the scalp of the individual, an eyelash of the individual, and an eyebrow hair of the individual.

32. The process of any one of claims 1 to 31, wherein the image scanner is associated with a first computer located (14) at a first location, and wherein the process further comprises transferring the scanned image data from the first computer to a second computer (20) located at a second location remote from the first location.

33. The process of claim 32, wherein the transferring includes transmitting the scanned image data via the Internet.

34. The process of claim 32 or 33, further comprising storing the scanned image data on a data storage medium, wherein the transferring includes shipping the data storage medium to the second location.

35. The process of any one of claims 32 to 34, further comprising transferring questionnaire answers from the first location to the second location, at least some of the questionnaire answers being related to at least one of the condition of the external portion and the product applied to the external portion

36. The process of any one of claims 32 to 35, further comprising displaying an image corresponding to the scanned image data, the displayed image including representations of the at least one characteristic, and wherein the analyzing includes viewing the displayed image.

37. The process of any one of claims 32 to 36, wherein the image is displayed at the second location.

38. The process of any one of claims 1 to 37, wherein a computer at least partially performs at least one of the analyzing and the determining.

39. The process of any one of claims 1 to 38, further comprising sending the scanned image data to a plurality of locations so that the at least one characteristic may be analyzed numerous times.

40. The process of any one of claims 1 to 39, further comprising
determining a recommendation of at least one cosmetic treatment for said at least one diagnosed condition of the external portion; and
providing the cosmetic treatment recommendation so that the external portion of the individual may treated according to the recommendation.

41. The process of claim 40, wherein the cosmetic treatment recommendation is a recommendation regarding use of a cosmetic product.

42. The process of claim 41, wherein the cosmetic product is one of a makeup product, a care product, a hair product, a skin product, and a sun exposure product.

43. The process of claim 41, wherein the treatment recommendation is a recommendation regarding application of said cosmetic product to the external portion.

44. The process of claim 40, wherein the providing of the treatment recommendation includes providing the treatment recommendation to at least one of the individual and a treatment provider via the Internet.

45. The process of any one of claims 40 to 44, wherein a computer at least partially performs the determining of the treatment recommendation, the computer being located at a location remote from that of the image scanner.

46. The process of any one of the preceding claims, further comprising monitoring status of the external portion during treatment for the diagnosed condition of the external portion.

47. The process of claim 46, further comprising providing a recommendation for an additional non medical treatment based on the monitored status.

48. The process of claim 47, further comprising providing the individual with information regarding the effectiveness of the treatment.

49. The process of claim 46, wherein the monitoring includes repeating at least the obtaining and the analyzing.

50. The process of any one of claims 1 to 49, further comprising collecting information relating to at least one of the scanned external portion and the product to form a database for use in at least one of diagnoses, treatment recommendation determinations, product evaluations, and product formulations.

51. The process of any one of the preceding claims, wherein the analyzing further comprising comparing an image formed from the scanned image data to at least one image formed from image data stored in an image database.

52. The process of any one of the preceding claims, further comprising providing the non medical diagnosis to at least one of the individual and a treatment provider via the Internet.

53. The process of any one of the preceding claims, wherein the scanned image data includes data regarding color of said at least one characteristic.

54. The process of any one of the preceding claims, wherein the obtaining of the scanned image data includes at least one of emitting light from the scanner onto the external portion, and emitting light from the scanner onto a transfer member.

55. The process of any one of the preceding claims, wherein the obtaining of the scanned information includes scanning, with the image scanner, a calibration member having one of a predetermined size and a predetermined color.

56. The process of any one of the preceding claims, wherein the obtaining includes obtaining scanned image data relating to multiple scanned images.

57. The process of any one of the preceding claims, wherein the process further comprises analyzing the external portion with analysis equipment.

58. The process of claim 57, wherein the analysis equipment is chosen from one of a corneometer, a dermal torque meter, an image analyzer, a PH meter, and a device for measuring hydration of the skin.

59. The process of any one of the preceding claims, further comprising providing a grade indicative of at least one of the condition of the external portion and performance of the product.

60. The process of claim 59, further comprising storing information relating to the grade in a database.

## Patentansprüche

1. Verfahren zum Diagnostizieren von einem oder mehreren Zuständen eines äußeren Körperteils und/oder einer oder mehreren Eigenschaften von wenigstens einem Produkt, das auf den äußeren Körperteil aufgetragen worden ist, das auf gescannter Information basiert, wobei das Verfahren umfasst:
Gewinnen von gescannten Bilddaten mit einem optischen Bildscanner (12), konfiguriert in der Form eines Scanners zum Scannen von Dokumenten, die sich auf wenigstens eines der folgenden Merkmale beziehen:
ein nicht den Fingerabdruck betreffenden äußeren Teil eines Individuums
und/oder
wenigstens ein Produkt, das auf den äußeren Teil aufgetragen ist; Analysieren des wenigstens einen Merkmals basierend auf den Bilddaten; und
Bestimmen einer nicht-medizinischen Diagnose von
wenigstens einem Zustand des äußeren Teils und/oder
wenigstens einer Eigenschaft des auf den äußeren Teil aufgetragenen Produktes.

2. Verfahren nach Anspruch 1, worin das Merkmal des äußeren Teils wenigstens eines einschließt von Falten, Krähenfüße, Blutgefäßnetze, die durch die Haut sichtbar sind, Hautporen, auf einen äußeren Körperteil aufgetragene kosmetische Materialien, sichtbare Eigenschaften von Haarsträhnen, einschließlich Wurzeln, sichtbare Eigenschaften von Haut, einschließlich Pigmentierungen und Gruppen aus Hautzellen; sichtbare Eigenschaften von Fingernägeln und Fußnägeln und von außen sichtbare Eigenschaften von Zähnen.

3. Verfahren nach Anspruch 1, worin das Merkmal des Produktes wenigstens eines einschließt von Nichtübertragbarkeit, Deckkraft des Produktes, Brillanz, Färbung, Fettigkeit, Wechselwirkungen zwischen der Haut und dem Produkt, Produktdicke und Produktmenge.

4. Verfahren nach Anspruch 1, worin das Gewinnen der gescannten Bilddaten das Platzieren des äußeren Teils des Individuums in der Nähe eines Scanbereichs des Scanners und Scannen des äußeren Teils mit dem Bildscanner, um die gescannten Bilddaten zu gewinnen, einschließt.

5. Verfahren nach Anspruch 4, worin der äußere Teil des Individuums in Kontakt mit dem Scanbereich des Scanners gebracht wird.

6. Verfahren nach Anspruch 5, worin der Scanner ein Flachbettscanner ist und worin der äußere Teil des Individuums bewegt wird, um in Kontakt mit dem Scanbereich zu gelangen.

7. Verfahren nach Anspruch 5, worin der Scanner ein in der Hand gehaltener Scanner ist und worin der Scanner bewegt wird, um in Kontakt mit dem äußeren Teil des Individuums zu gelangen.

8. Verfahren nach Anspruch 4, weiterhin umfassend das Einbringen von Flüssigkeit zwischen dem äußeren Körperteil und dem Scanbereich, wobei die Flüssigkeit den Brechungsindex ändert, um das Betrachten des wenigstens einen Merkmals zu verbessern.

9. Verfahren nach Anspruch 4, weiterhin umfassend das Einbringen von wenigstens einem Farbstoff und einem Pigment auf den äußeren Teil, um das Betrachten des wenigstens einen Merkmals zu verbessern.

10. Verfahren nach Anspruch 4, worin das Gewinnen der gescannten Bilddaten weiterhin das In-Kontakt-Bringen eines Übertragungsgliedes mit dem äußeren Teil des Individuums einschließt, um ein Bild auf dem Übertragungsglied bereitzustellen, und worin das Scannen weiterhin das Scannen des Bildes des Übertragungsgliedes mit dem Scanner einschließt.

11. Verfahren nach Anspruch 10, worin das Scannen des äußeren Teils und das Scannen des Bildes des Übertragungsgliedes eines von gleichzeitig und nacheinander geschehen.

12. Verfahren nach Anspruch 1, worin das Gewinnen der gescannten Bilddaten das In-Kontakt-Bringen eines Übertragungsgliedes mit dem äußeren Teil des Individuums einschließt, um ein Bild des Übertragungsgliedes bereitzustellen, und Scannen des Bildes des Übertragungsgliedes mit dem Scanner, um die gescannten Bilddaten zu gewinnen.

13. Verfahren nach Anspruch 12, worin das Übertragungsglied Klebematerial einschließt, bereitgestellt auf einer Trägerschicht, wobei das Klebematerial des Übertragungsgliedes in Kontakt mit Haut gebracht wird und das Übertragungsglied von der Haut entfernt wird, um Zellen von der Haut des Individuums auf das Übertragungsglied zu übertragen.

14. Verfahren nach Anspruch 13, worin das Analysieren das Beurteilen der Menge an Zellen, die auf das Übertragungsglied übertragen worden sind, einschließt und worin der diagnostizierte Zustand die Trockenheit der Haut ist.

15. Verfahren nach Anspruch 13, weiterhin umfassend das In-Kontakt-Bringen des Klebematerials des Übertragungsgliedes mit Klebematerial eines zweiten Übertragungsgliedes und Trennen der Übertragungsglieder, um einen Teil der Hautzellen auf das zweite Übertragungsglied zu übertragen.

16. Verfahren nach Anspruch 12, worin das Übertragungsglied in Kontakt mit einem äu-ßeren Körperteil gebracht wird, einschließlich eines darauf aufgetragenen kosmetischen Produktes, und worin ein aus den eingescannten Bilddaten gebildetes Bild repräsentativ für wenigstens ein Merkmal des kosmetischen Produktes ist.

17. Verfahren nach Anspruch 16, worin der äußere Teil Lippen einschließt und worin das kosmetische Produkt eines von einem Lippenpflegeprodukt und einem Lippen-Make-up-Produkt ist.

18. Verfahren nach Anspruch 16, worin der äußere Teil Haut einschließt und worin das kosmetische Produkt Grundierungs-Make-up ist.

19. Verfahren nach Anspruch 18, worin das Übertragungsglied aus Gewebe gebildet ist.

20. Verfahren nach Anspruch 19, worin das Übertragungsglied ein Bekleidungsartikel ist.

21. Verfahren nach Anspruch 12, worin das Übertragungsglied ein Blatt aus einem Material ist und worin das Verfahren das In-Kontakt-Bringen des Blattes aus einem Material mit Lippen des Individuums und Übertragen eines Lippenproduktes von den Lippen auf das Blatt aus einem Material einschließt.

22. Verfahren nach Anspruch 21, worin das Lippenprodukt Lippenstift ist und wobei die diagnostizierte Eigenschaft das Nichthaften des Lippenstiftes auf den Lippen ist.

23. Verfahren nach Anspruch 12, worin das Übertragungsglied ein formbares Material ist und worin das Verfahren das In-Kontakt-Bringen des formbaren Materials mit der Haut des Individuums einschließt, um auf dem formbaren Material das Oberflächenprofil der Haut zu erzeugen.

24. Verfahren nach Anspruch 12, worin das Übertragungsglied ein Fenster ist, wobei das Fenster ein Teil des Bildscanners ist, der einen Scanbereich definiert.

25. Verfahren nach Anspruch 12, worin das Übertragungsglied eines ist aus einem Haarkamm und einer Haarbürste, worin das In-Kontakt-Bringen des Übertragungsgliedes mit dem äußeren Körperteil das Hindurchführen dieses einen aus dem Haarkamm und der Haarbürste durch das Haar einschließt, und worin das Bild auf dem Übertragungsglied wenigstens eines aus Haarsträhne und Hautzellen einschließt.

26. Verfahren nach Anspruch 12, worin das Bild auf dem Übertragungsglied einen Zustand des äußeren Teils anzeigt.

27. Verfahren nach Anspruch 12, worin das Übertragungsglied so gebaut ist, dass es die Farbe in Antwort auf einen Zustand des äußeren Teils ändert.

28. Verfahren nach Anspruch 1, worin der äußere Teil wenigstens eines der Haut des Individuums, wenigstens eine Strähne des Haars des Individuums, wenigstens einen Fingernagel des Individuums, wenigstens einen Zehnagel des Individuums und wenigstens einen Zahn des Individuums einschließt.

29. Verfahren nach Anspruch 28, worin der äußere Teil die Haut des Individuums einschließt und worin der äußere Teil auf einem von der Hand, dem Fuß, Arm, Bein, Torso und Gesicht des Individuums lokalisiert ist.

30. Verfahren nach Anspruch 29, worin der äußere Teil auf den Lippen des Individuums lokalisiert ist.

31. Verfahren nach Anspruch 28, worin der äußere Teil diese wenigstens eine Haarsträhne einschließt und worin diese wenigstens eine Haarsträhne eine ist einer Haarsträhne von der Kopfhaut des Individuums, einer Wimper des Individuums und einem Augenbrauenhaar des Individuums.

32. Verfahren nach einem der Ansprüche 1 bis 31, worin der Bildscanner mit einem ersten Computer in Verbindung steht, der an einer ersten Örtlichkeit lokalisiert ist (14), und worin das Verfahren weiterhin das Übertragen der gescannten Bilddaten von dem ersten Computer zu einem zweiten Computer (20) umfasst, lokalisiert an einer zweiten Örtlichkeit, entfernt von der ersten Örtlichkeit.

33. Verfahren nach Anspruch 32, worin das Übertragen das Übermitteln der gescannten Bilddaten über das Internet einschließt.

34. Verfahren nach Anspruch 32 oder 33, weiter umfassend das Speichern der gescannten Bilddaten auf einem Datenspeichermedium, worin das Übertragen das Verbringen des Datenspeichermediums an die zweite Örtlichkeit einschließt.

35. Verfahren nach irgendeinem der Ansprüche 32 bis 34, weiterhin umfassend das Übertragen von Fragebogenantworten von der ersten Örtlichkeit zu der zweiten Örtlichkeit, wobei wenigstens einige der Fragebogenantworten in Zusammenhang stehen mit wenigstens einem Zustand des äußeren Teils und des auf den äußeren Teil aufgetragenen Produktes.

36. Verfahren nach irgendeinem der Ansprüche 32 bis 35, weiterhin umfassend das Zeigen eines den gescannten Bilddaten entsprechenden Bildes, wobei das gezeigte Bild Darstellungen des wenigstens einen Merkmals einschließt und worin das Analysieren das Betrachten des gezeigten Bildes einschließt.

37. Verfahren nach irgendeinem der Ansprüche 32 bis 35, worin das Bild an der zweiten Örtlichkeit gezeigt wird.

38. Verfahren nach irgendeinem der Ansprüche 1 bis 37, worin ein Computer wenigstens teilweise wenigstens eines von dem Analysieren und dem Bestimmen durchführt.

39. Verfahren nach irgendeinem der Ansprüche 1 bis 38, weiterhin umfassend das Senden der gescannten Bilddaten an eine Vielzahl von Örtlichkeiten, so dass das wenigstens eine Merkmal zahlreiche Male analysiert werden kann.

40. Verfahren nach irgendeinem der Ansprüche 1 bis 39, weiterhin umfassend das Bestimmen einer Empfehlung von wenigstens einer kosmetischen Behandlung für diesen wenigstens einen diagnostizierten Zustand des äußeren Teils; und Bereitstellen der kosmetischen Behandlungsempfehlung, so dass der äußere Teil des Individuums gemäß der Empfehlung behandelt werden kann.

41. Verfahren nach Anspruch 40, worin die kosmetische Behandlungsempfehlung eine Empfehlung im Hinblick auf die Verwendung eines kosmetischen Produktes ist.

42. Verfahren nach Anspruch 41, worin das kosmetische Produkt eines ist aus einem Make-up-Produkt, einem Pflegeprodukt, einem Haarprodukt, einem Hautprodukt und einem Sonnenaussetzungsprodukt.

43. Verfahren nach Anspruch 41, worin die Behandlungsempfehlung eine Empfehlung im Hinblick auf das Auftragen dieses kosmetischen Produktes auf den äußeren Teil ist.

44. Verfahren nach Anspruch 40, worin das Bereitstellen der Behandlungsempfehlung das Bereitstellen der Behandlungsempfehlung für wenigstens einen von dem Individuum und einem Behandlungsbereitsteller über das Internet einschließt.

45. Verfahren nach irgendeinem der Ansprüche 40 bis 44, worin ein Computer wenigstens teilweise das Bestimmen der Behandlungsempfehlung durchführt, wobei der Computer an einer Örtlichkeit lokalisiert ist, die von jener des Bildscanners entfernt ist.

46. Verfahren nach irgendeinem der vorstehenden Ansprüche, weiterhin umfassend das Überwachen des Zustands des äußeren Teils während der Behandlung für den diagnostizierten Zustand des äußeren Teils.

47. Verfahren nach Anspruch 46, weiterhin umfassend das Bereitstellen einer Empfehlung für eine zusätzliche nicht-medizinische Behandlung, basierend auf dem überwachten Zustand.

48. Verfahren nach Anspruch 47, weiterhin umfassend das Versorgen des Individuums mit Information im Hinblick auf die Wirksamkeit der Behandlung.

49. Verfahren nach Anspruch 46, worin das Überwachen das Wiederholen von wenigstens der Gewinnung und der Analysierung einschließt.

50. Verfahren nach irgendeinem der Ansprüche 1 bis 49, weiterhin umfassend das Sammeln von Information in Bezug auf wenigstens eines des gescannten äußeren Teils und des Produktes, um eine Datenbank zur Verwendung bei wenigstens einem von Diagnosen, Behandlungsempfehlungsbestimmungen, Produktbeurteilungen und Produktformulierungen zu bilden.

51. Verfahren nach irgendeinem der vorstehenden Ansprüche, worin das Analysieren weiter das Vergleichen eines aus den eingescannten Bilddaten gebildeten Bildes mit wenigstens einem aus den in einer Bilddatenbank gelagerten Bilddaten gebildeten Bildes umfasst.

52. Verfahren nach irgendeinem der vorstehenden Ansprüche, weiterhin umfassend das Bereitstellen der nicht-medizinischen Diagnose an wenigstens einen von dem Individuum und dem Behandlungsbereitsteller über das Internet.

53. Verfahren nach irgendeinem der vorstehenden Ansprüche, worin die eingescannten Bilddaten Daten im Hinblick auf Farbe dieses wenigstens einen Merkmals einschließen.

54. Verfahren nach irgendeinem der vorstehenden Ansprüche, worin das Gewinnen der gescannten Bilddaten wenigstens eines von Emittieren von Licht von dem Scanner auf den äußeren Teil und Emittieren von Licht von dem Scanner auf ein Übertragungsglied einschließt.

55. Verfahren nach irgendeinem der vorstehenden Ansprüche, worin das Gewinnen der gescannten Information das Scannen eines Kalibrierungsgliedes, welches eines aufweist von einer vorbestimmten Größe und einer vorbestimmten Farbe, mit dem Bildscanner einschließt.

56. Verfahren nach irgendeinem der vorstehenden Ansprüche, worin das Gewinnen das Gewinnen von gescannten Bilddaten in Bezug auf mehrere gescannte Bilder einschließt.

57. Verfahren nach irgendeinem der vorstehenden Ansprüche, worin das Verfahren weiter umfasst das Analysieren des äußeren Teils mit Analyseausrüstung.

58. Verfahren nach Anspruch 57, worin die Analyseausrüstung ausgewählt wird aus einem von einem Corneometer, einem Hautmomentmessgerät, einem Bildanalysegerät, einem pH-Meter und einer Vorrichtung zum Messen der Feuchtigkeit der Haut.

59. Verfahren nach irgendeinem der vorstehenden Ansprüche, weiterhin umfassend das Bereitstellen eines Grades, der anzeigend ist für wenigstens eines von dem Zustand des äußeren Teils und dem Leistungswert des Produktes.

60. Verfahren nach Anspruch 59, weiterhin umfassend das Speichern von Information in Bezug auf den Grad in einer Datenbank.

## Revendications

1. Procédé pour diagnostiquer, sur la base d'informations scannées, une ou plusieurs conditions d'une partie de corps externe et/ou une ou plusieurs propriétés d'au moins un produit appliqué sur la partie de corps externe, le procédé comportant les étapes consistant à :
obtenir, à l'aide d'un scanner d'image optique (12) configuré sous la forme d'un dispositif pour scanner des documents, des données d'image scannées concernant au moins une caractéristique de
une partie externe non-dermatoglyphique d'un individu et/ou
au moins un produit appliqué sur la partie externe,
analyser, sur la base des données d'image, la au moins une caractéristique, et
déterminer un diagnostic non-médical de
au moins une condition de la partie externe, et/ou
au moins une propriété du produit appliqué sur la partie externe.

2. Procédé selon la revendication 1, dans lequel les caractéristiques de la partie externe incluent au moins une caractéristique parmi des rides, des pattes d'oie, des réseaux de vaisseaux sanguins visibles à travers la peau, des pores de la peau, des matières cosmétiques appliqués à une partie de corps externe, des propriétés visibles de brins de cheveux incluant des racines, des propriétés visibles de la peau incluant des pigmentations et des groupes de cellules de la peau, des propriétés visibles d'ongles des doigts et d'ongles des pieds, et des propriétés extérieurement visibles des dents.

3. Procédé selon la revendication 1, dans lequel la caractéristique du produit inclut au moins une caractéristique parmi une non-transférabilité, une couverture de produit, une brillance, une coloration, le caractère gras, des interactions entre la peau et le produit, une épaisseur de produit, et une quantité de produit.

4. Procédé selon la revendication 1, dans lequel l'obtention des données d'image scannées inclut la mise en place de la partie externe de l'individu à proximité d'une région de balayage du scanner, et le balayage de la partie externe à l'aide du scanner pour obtenir les données d'image scannées.

5. Procédé selon la revendication 4, dans lequel la partie externe de l'individu est placée en contact avec la région de balayage du scanner.

6. Procédé selon la revendication 5, dans lequel le scanner est un scanner à plat et dans lequel la partie externe de l'individu est déplacée en contact avec la région de balayage.

7. Procédé selon la revendication 5, dans lequel le scanner est un scanner portatif et dans lequel le scanner est déplacé en contact avec la partie externe de l'individu.

8. Procédé selon la revendication 4, comportant de plus la mise en place d'un liquide entre la partie de corps externe et la région de balayage, le liquide altérant l'indice de réfraction pour améliorer l'observation de la au moins une caractéristique.

9. Procédé selon la revendication 4, comportant de plus la mise en place d'au moins un parmi un colorant et un pigment sur la partie externe pour améliorer l'observation de la au moins une caractéristique.

10. Procédé selon la revendication 4, dans lequel l'obtention des données d'image scannées inclut de plus la mise en place d'un élément de transfert en contact avec la partie externe de l'individu pour fournir une image sur l'élément de transfert, et dans lequel le balayage inclut de plus le balayage de l'image de l'élément de transfert à l'aide du scanner.

11. Procédé selon la revendication 10, dans lequel le balayage de la partie externe et le balayage de l'image de l'élément de transfert ont lieu simultanément et l'un après l'autre.

12. Procédé selon la revendication 1, dans lequel l'obtention des données d'image scannées inclut la mise en place d'un élément de transfert en contact avec la partie externe de l'individu pour fournir une image sur l'élément de transfert, et le balayage de l'image de l'élément de transfert à l'aide du scanner pour obtenir les données d'image scannées.

13. Procédé selon la revendication 12, dans lequel l'élément de transfert inclut un matériau adhésif agencé sur un élément de renfort, le matériau adhésif de l'élément de transfert étant placé en contact avec la peau et l'élément de transfert étant retiré de la peau pour transférer des cellules depuis la peau de l'individu sur l'élément de transfert.

14. Procédé selon la revendication 13, dans lequel l'analyse inclut l'évaluation de la quantité de cellules transférées sur l'élément de transfert et dans lequel la condition diagnostiquée est la siccité de la peau.

15. Procédé selon la revendication 13, comportant de plus la mise en place du matériau adhésif de l'élément de transfert en contact avec le matériau adhésif d'un second élément de transfert et la séparation des éléments de transfert pour transférer une partie des cellules de la peau sur le second élément de transfert.

16. Procédé selon la revendication 12, dans lequel l'élément de transfert est placé en contact avec une partie de corps externe incluant un produit cosmétique appliqué dessus, et dans lequel une image formée à partir des données d'image scannées est représentative d'au moins une caractéristique du produit cosmétique.

17. Procédé selon la revendication 16, dans lequel la partie externe inclut des lèvres et dans lequel le produit cosmétique est l'un parmi un produit de soin des lèvres et un produit de maquillage des lèvres.

18. Procédé selon la revendication 16, dans lequel la partie externe inclut la peau et dans lequel le produit cosmétique est un fond de teint.

19. Procédé selon la revendication 18, dans lequel l'élément de transfert est formé d'un tissu.

20. Procédé selon la revendication 19, dans lequel l'élément de transfert est un article de vêtement.

21. Procédé selon la revendication 12, dans lequel l'élément de transfert est une feuille de matériau, et dans lequel le processus inclut la mise en place de la feuille de matériau en contact avec des lèvres de l'individu et le transfert d'un produit pour lèvres depuis les lèvres sur la feuille de matériau.

22. Procédé selon la revendication 21, dans lequel le produit pour lèvres est un rouge à lèvres, la propriété diagnostiquée étant la non-rétention du rouge à lèvres sur les lèvres.

23. Procédé selon la revendication 12, dans lequel l'élément de transfert est un matériau moulable, et dans lequel le procédé inclut la mise en place du matériau moulable en contact avec la peau de l'individu pour produire, sur le matériau moulable, le profil de surface de la peau.

24. Procédé selon la revendication 12, dans lequel l'élément de transfert est une vitre, la vitre étant une partie du scanner définissant une région de balayage.

25. Procédé selon la revendication 12, dans lequel l'élément de transfert est l'un parmi un peigne à cheveux et une brosse à cheveux, dans lequel la mise en place de l'élément de transfert en contact avec la partie de corps externe inclut le passage dudit au moins l'un parmi le peigne à cheveux et la brosse à cheveux à travers les cheveux, et dans lequel l'image sur l'élément de transfert inclut au moins un parmi des brins de cheveux et des cellules de la peau.

26. Procédé selon la revendication 12, dans lequel l'image sur l'élément de transfert indique une condition de la partie externe.

27. Procédé selon la revendication 12, dans lequel l'élément de transfert est configuré pour changer de couleur en réponse à une condition de la partie externe.

28. Procédé selon la revendication 1, dans lequel la partie externe inclut au moins une partie parmi la peau de l'individu, au moins un brin de cheveu de l'individu, au moins un ongle des doigts de l'individu, au moins un ongle des pieds de l'individu, et au moins une dent de l'individu.

29. Procédé selon la revendication 28, dans lequel la partie externe inclut la peau de l'individu, et dans lequel la partie externe est positionnée sur l'un parmi la main, le pied, le bras, la jambe, le torse et le visage de l'individu.

30. Procédé selon la revendication 29, dans lequel la partie externe est située sur les lèvres de l'individu.

31. Procédé selon la revendication 28, dans lequel la partie externe inclut ledit au moins un brin de cheveu, et dans lequel ledit au moins un brin de cheveu est l'un parmi un brin de cheveu provenant du cuir chevelu de l'individu, un cil de l'individu, et un sourcil de l'individu.

32. Procédé selon l'une quelconque des revendications 1 à 31, dans lequel le scanner est associé à un premier ordinateur (14) situé à un premier emplacement, et dans lequel le procédé comporte de plus le transfert des données d'image scannées depuis le premier ordinateur dans un second ordinateur (20) situé à un second emplacement éloigné du premier emplacement.

33. Procédé selon la revendication 32, dans lequel le transfert inclut la transmission des données d'image scannées via l'Internet.

34. Procédé selon la revendication 32 ou 33, comportant de plus la mémorisation des données d'image scannées sur un support de mémorisation de données, et dans lequel le transfert inclut le transport du support de mémorisation de données jusqu'au second emplacement.

35. Procédé selon l'une quelconque des revendications 32 à 34, comportant de plus le transfert de réponses à un questionnaire depuis le premier emplacement jusqu'au second emplacement, au moins une partie des réponses au questionnaire étant associée à au moins une des conditions de la partie externe et au produit appliqué sur la partie externe.

36. Procédé selon l'une quelconque des revendications 32 à 35, comportant de plus l'affichage d'une image correspondant aux données d'image scannées, l'image affichée incluant des représentations de la au moins une caractéristique, et dans lequel l'analyse inclut l'observation de l'image affichée.

37. Procédé selon l'une quelconque des revendications 32 à 36, dans lequel l'image est affichée au second emplacement.

38. Procédé selon l'une quelconque des revendications 1 à 37, dans lequel un ordinateur effectue au moins partiellement au moins l'une parmi une analyse et une détermination.

39. Procédé selon l'une quelconque des revendications 1 à 38, comportant de plus l'émission de données d'image scannées vers une pluralité d'emplacements de sorte que la au moins une caractéristique peut être analysée de nombreuses fois.

40. Procédé selon l'une quelconque des revendications 1 à 39, comportant de plus
la détermination d'une recommandation d'au moins un traitement cosmétique pour ladite au moins une condition diagnostiquée de la partie externe, et
la fourniture de la recommandation de traitement cosmétique de sorte que la partie externe de l'individu peut être traitée conformément à la recommandation.

41. Procédé selon la revendication 40, dans lequel la recommandation de traitement cosmétique est une recommandation concernant l'utilisation d'un produit cosmétique.

42. Procédé selon la revendication 41, dans lequel le produit cosmétique est l'un parmi un produit de maquillage, un produit de soin, un produit pour cheveux, un produit pour la peau, et un produit pour l'exposition au soleil.

43. Procédé selon la revendication 41, dans lequel la recommandation de traitement est une recommandation concernant l'application dudit produit cosmétique sur la partie externe.

44. Procédé selon la revendication 40, dans lequel la fourniture de la recommandation de traitement inclut la fourniture de la recommandation de traitement à au moins l'un parmi l'individu et un fournisseur de traitement via l'Internet.

45. Procédé selon l'une quelconque des revendications 40 à 44, dans lequel un ordinateur effectue au moins partiellement la détermination de la recommandation de traitement, l'ordinateur étant situé à un emplacement éloigné de celui du scanner.

46. Procédé selon l'une quelconque des revendications précédentes, comportant de plus la surveillance d'état de la partie externe pendant le traitement pour la condition diagnostiquée de la partie externe.

47. Procédé selon la revendication 46, comportant de plus la fourniture d'une recommandation pour un traitement non-médical supplémentaire basé sur l'état surveillé.

48. Procédé selon la revendication 47, comportant de plus la fourniture à l'individu d'informations concernant l'efficacité du traitement.

49. Procédé selon la revendication 46, dans lequel la surveillance inclut la répétition au moins de l'obtention et de l'analyse.

50. Procédé selon l'une quelconque des revendications 1 à 49, comportant de plus la collecte d'informations concernant au moins l'un parmi la partie externe balayée et le produit pour former une base de données destinée à être utilisée dans au moins l'un parmi des diagnostics, des déterminations de recommandation de traitement, des évaluations de produit, et des formulations de produit.

51. Procédé selon l'une quelconque des revendications précédentes, dans lequel l'analyse comporte de plus la comparaison d'une image formée à partir des données d'image scannées avec au moins une image formée à partir de données d'image mémorisées dans une base de données d'images.

52. Procédé selon l'une quelconque des revendications précédentes, comportant de plus la fourniture du diagnostic non-médical à au moins l'un parmi l'individu et un fournisseur de traitement via l'Internet.

53. Procédé selon l'une quelconque des revendications précédentes, dans lequel les données d'image scannées incluent des données concernant une couleur de ladite au moins caractéristique.

54. Procédé selon l'une quelconque des revendications précédentes, dans lequel l'obtention des données d'image scannées inclut au moins l'une parmi une émission de lumière depuis le scanner sur la partie externe, et une émission de lumière depuis le scanner sur un élément de transfert.

55. Procédé selon l'une quelconque des revendications précédentes, dans lequel l'obtention des informations scannées inclut le balayage, à l'aide du scanner, d'un élément d'étalonnage ayant l'une parmi une taille prédéterminée et une couleur prédéterminée.

56. Procédé selon l'une quelconque des revendications précédentes, dans lequel l'obtention inclut l'obtention de données d'image scannées concernant de multiples images scannées.

57. Procédé selon l'une quelconque des revendications précédentes, dans lequel le procédé comporte de plus l'analyse de la partie externe à l'aide d'un équipement d'analyse.

58. Procédé selon la revendication 57, dans lequel l'équipement d'analyse est choisi parmi un cornéomètre, un dispositif de mesure de couple dermique, un analyseur d'image, un pH-mètre, et un dispositif pour mesurer l'hydratation de la peau.

59. Procédé selon l'une quelconque des revendications précédentes, comportant de plus la fourniture d'une qualité représentative d'au moins l'une parmi la condition de la partie externe et la performance du produit.

60. Procédé selon la revendication 59, comportant de plus la mémorisation d'informations concernant la qualité dans une base de données.
